Europäisches Patentamt

European Patent Office

Office européen des brevets

(19)

(11) Publication number: **0 164 939**
**B1**

(12) **EUROPEAN PATENT SPECIFICATION**

(45) Date of publication of patent specification: **14.09.88**　　(51) Int. Cl.⁴: **C 01 B 33/28**

(21) Application number: **85303605.1**

(22) Date of filing: **22.05.85**

(54) **Preparation of zeolite beta.**

(30) Priority: **11.06.84 US 619529**

(43) Date of publication of application:
**18.12.85 Bulletin 85/51**

(45) Publication of the grant of the patent:
**14.09.88 Bulletin 88/37**

(84) Designated Contracting States:
**BE DE FR GB IT NL SE**

(56) References cited:
**EP-A-0 015 702**
**EP-A-0 055 046**
**EP-A-0 064 577**
**FR-A-2 429 182**

(73) Proprietor: **MOBIL OIL CORPORATION**
**150 East 42nd Street**
**New York New York 10017 (US)**

(72) Inventor: **Calvert, Robert Bruce**
**157 Hayward Mill Road**
**Concord, Mass. 01742 (US)**
Inventor: **Valyocsik, Ernest William**
**960 Randolph Drive**
**Yardley, PA 19067 (US)**
Inventor: **Wong, Stephen Sui Fai**
**3 Carrol Joy Road**
**Medford, N.J. 08055 (US)**

(74) Representative: **Carpmael, John William**
**Maurice et al**
**CARPMAELS & RANSFORD 43 Bloomsbury**
**Square**
**London, WC1A 2RA (GB)**

EP 0 164 939 B1

Courier Press, Leamington Spa, England.

## Description

This invention relates to an improved method of preparing a partially crystalline zeolite Beta composition whereby synthesis is facilitated and of reduced cost and the product exhibits an extended high silica/alumina mole ratio when compared to conventionally synthesized zeolite Beta without the need for dealumination. The new composition prepared hereby shows surprisingly high activity and selectivity in organic compound conversion, notably in catalytic hydroisomerization processing.

Crystalline zeolite Beta, which is identified by its x-ray diffraction pattern, and its conventional preparation are disclosed in US—A—3,308,069.

A zeolite having the structure of zeolite Beta is described in EP—A—55046 ("NU-2") and in GB—A—2,024,790 ("Borallite B"). Zeolite other than zeolite Beta are shown to be prepared from various reaction mixtures having various silica/alumina reaction mixture mole ratios in US—A—4,366,135; 4,297,335; 4,275,047 and 4,257,885. Preparation of zeolite ZSM-5 having a $SiO_2$ content of more than 90% by weight is disclosed in EP—A—40444. EP—A—36683 and GB—A—1,553,209 disclose reaction mixtures useful for preparing zeolites other than zeolite Beta.

According to the invention a method for preparing zeolite Beta comprises forming a reaction mixture containing sources of alkali metal oxide, an organic nitrogen-containing cation, an oxide of aluminum, an oxide of silicon and water and having a composition, in terms of mole ratios, within the following ranges:

|  | Broad | Preferred |
|---|---|---|
| $SiO_2/Al_2O_3$ | 20—1000 | 30—500 |
| $H_2O/SiO_2$ | 5—200 | 10—100 |
| $OH^-/SiO_2$ | 0.10—2.0 | 0.2—1.0 |
| $M/SiO_2$ | 0.01—1.0 | 0.05—0.20 |
| $R/SiO_2$ | 0.10—2.0 | 0.20—1.0 |

wherein R is a cation derived from a tetraethylammonium compound and M is an alkali metal ion, and maintaining the mixture for from about 24 hours to about 7 days until crystallinity of the product zeolite Beta is from about 30% to about 90%. Crystallization is purposely terminated at a time which provides only from about 30 to about 90% crystallinity. Thereafter, the partially crystalline zeolite Beta is separated from the liquid and recovered. Reaction conditions required consist of heating the foregoing reaction mixture to a temperature of from about 90°C to about 200°C for a period of time of from about 24 hours to about 7 days. A more preferred temperature range is from about 150°C to about 160°C with the amount of time at a temperature in such range being from about 48 hours to about 5 days.

The digestion of the gel particles is carried out until zeolite Beta is formed having crystallinity from about 30% to about 90%. Crystallization is then terminated and the solid product separated from the reaction medium, as by cooling the whole to room temperature, filtering and water washing.

The key variables in the above reaction mixture composition are the molar ratios of $OH^-/SiO_2$ and $M/SiO_2$. If $OH^-/SiO_2$ is not optimized within the range of 0.10 to 2.0, preferably at least 0.5, and $M/SiO_2$ is not less than 1.0, preferably less than 0.2, product other than partially crystalline Beta, e.g. ZSM-5 and ZSM-12, will result. The value of the ratio $SiO_2/Al_2O_3$ is preferably greater than 200.

Fully crystalline zeolite Beta synthesized under conventional procedure is proven to have catalytic application, but the range of product silica/alumina mole ratio is relatively limited. When the partially crystalline composition of zeolite Beta is synthesized in accordance with the present method, it exhibits silica/alumina mole ratios over a wide and higher range and significantly enhanced catalytic activity for certain conversions of interest, including hydroisomerization and hydrocracking. The present method allows the reaction mixture of silica, alumina and tetraethylammonium compound to produce the desired partially crystalline zeolite Beta.

The partially crystalline composition prepared hereby functions as well as fully crystalline zeolite Beta resulting in a cost savings in catalyst manufacture and needs no binder for certain applications, such as, for example, use as catalyst in hydroisomerization. Thus it is a less expensive catalyst than zeolites requiring binder, such as fully crystalline zeolite Beta.

The reaction mixture composition can be prepared from materials which supply the appropriate oxide. Such compositions include aluminates, alumina, silicates, silica hydrosol, silica gel, silicic acid and hydroxides. Each oxide component utilized in the reaction mixture for preparing the zeolite can be supplied by one or more essential reactants and they can be mixed together in any order. For example, any oxide can be supplied by an aqueous solution, sodium hydroxide or by an aqueous solution of a suitable silicate; the organic cation can be supplied by the directing agent compound of that cation, such as, for example, the hydroxide or a salt, e.g. halide, such as chloride, bromide or iodide. The reaction mixture can be prepared either batchwise or continuously. Crystal size and crystallization time will vary with the nature of the reaction mixture employed.

The tetraethylammonium compound may be, as non-limiting examples, the hydroxide or a halide, e.g. bromide.

The zeolite Beta composition freshly synthesized in accordance with the invention will usually conform with the formula in terms of mole ratios of oxides and in the anhydrous state:

$$(0.1 \text{ to } 4) \ R_2O: (0.05 \text{ to } 2) \ M_{2/n}O: (0.1 \text{ to } 5) \ Al_2O_3: (100) \ SiO_2$$

wherein M is at least one cation having a valence n, R is the cation derived from a tetraethylammonium compound, above described.

The original cations can be replaced, at least in part, by calcination and/or ion exchange with another cation. Thus, the original alkali metal cations are exchanged into a hydrogen or hydrogen ion precursor form or a form in which the original cation has been replaced by a metal of Groups IIA, IIIA, IVA, IB, IIB, IIIB, IVB, VIB or VIII of the Periodic Table. Thus, for example, it is contemplated to exchange the original cations with ammonium ions or with hydronium ions. Catalytically active forms of these would include, in particular, hydrogen, rare earth metals, aluminum, metals of Groups II and VIII of the Periodic Table and manganese.

The x-ray diffraction pattern of zeolite Beta has the characteristic lines shown in Table 1. The partially crystalline Beta prepared hereby provides the same pattern.

TABLE 1

| Interplanar d-spacing (A) | Relative intensity ($I/I_o$) |
|---|---|
| 11.5 ±0.3 | M-S |
| 7.4 ±0.2 | W |
| 6.6 ±0.15 | W |
| 4.15±0.1 | W |
| 3.97±0.1 | VS |
| 3.00±0.07 | W |
| 2.05±0.05 | W |

These values were determined by standard technique. The radiation was the K-alpha doublet of copper, and a diffraction equipped with a scintillation counter and a strip chart pen recorder was used. The peak heights, I, and the positions as a function of two times theta, where theta is the Bragg angle, were read from the spectometer chart. From these, the relative intensities, 100 $I/I_o$ where $I_o$ is the intensity of the strongest line or peak, and d (obs.), the interplanar spacing in Angstrom units (A) corresponding to the recorded lines, were calculated. In Table 1 the relative intensities are given in terms of the symbols W=weak, M=medium, S=strong and VS=very strong. Ion exchanged forms of the zeolite manifest substantially the same pattern with some minor shifts in interplanar spacing and variation in relative intensity. Other minor variations can occur depending on the silicon to aluminum ratio of the particular sample and its thermal history.

While the improved partially crystalline composition of the present invention may be used in a wide variety of organic compound, e.g. hydrocarbon compound, conversion reactions, it is notably useful in the processes of dewaxing, hydroisomerization and cracking. Other conversion processes for which partially crystalline zeolite Beta may be utilized in one or more of its active forms include, for example, hydrocracking and converting light aliphatics to aromatics such as in US—A—3,760,024.

Partially crystalline zeolite Beta prepared in accordance herewith can be used either in the organic nitrogen-containing the alkali metal containing form, the alkali metal form and hydrogen form or another univalent or multivalent cationic form. It can also be used in intimate combination with a hydrogenating component such as tungsten, vanadium, molybdenum, rhenium, nickel, cobalt, chromium, manganese, or a noble metal such as platinum or palladium where a hydrogenation-dehydrogenation function is to be performed. Such components can be exchanged into the composition, impregnated therein or physically intimately admixed therewith, for example by, in the case of platinum, treating the zeolite with a platinum metal-containing ion. Suitable platinum compounds for this purpose include chloroplatinic acid, platinous chloride and various compounds containing the platinum amine complex. Combinations of metals and methods for their introduction can also be used.

Partially crystalline zeolite Beta, when employed either as an adsorbent or as a catalyst, should be dehydrated at least partially, suitably by heating to a temperature in the range of from about 200°C to about 600°C in an inert atmosphere, such as air, nitrogen, etc. and at atmospheric or subatmospheric pressures for between 1 and 48 hours. Dehydration can also be performed at lower temperature merely by placing the zeolite in a vacuum, but a longer time is required to obtain a particular degree of dehydration. A thermal decomposition product of this material can be created by heating same at a temperature of up to about 600°C in an inert atmosphere for from about 1 hour to about 48 hours.

As above mentioned, partially crystalline zeolite Beta prepared in accordance herewith can have the original cations associated therewith replaced by a wide variety of other cations according to techniques well known in the art. Typical replacing cations include hydrogen, ammonium and metal cations including mixtures thereof. Of the replacing metallic cations, particular preference is given to cations of metals such as rare earths, Mn, Ca, Mg, Zn, Cd, Pd, Ni, Cu, Ti, Al, Sn, Fe and Co.

Typical ion exchange technique would be to contact the synthetic zeolite with a salt of the desired replacing cation or cations. Although a wide variety of salts can be employed, particular preference is given to chlorides, nitrates and sulfates.

Catalysts comprising zeolite Beta prepared according to the invention may be formed in a wide variety of particle sizes. Generally speaking, the particles can be in the form of a powder, a granule, or a molded product, such as extrudate having particle size sufficient to pass through a 2 mesh (Tyler) screen and be retained in a 400 mesh (Tyler) screen. In cases where the catalyst is molded, such as by extrusion, the crystalline silicate can be extruded before drying or dried or partially dried and then extruded.

Although not necessary for most applications, when it is desired to composite the partially crystalline zeolite Beta hereby prepared with another material, active and inactive materials and synthetic or naturally occurring zeolites as well as inorganic materials such as clays, silica and/or metal oxides, e.g. alumina, may be used, as described in our EP—A—1695.

Employing a catalytically active form of the composition of this invention containing a hydrogenation component, reforming stocks can be reformed employing a temperature between 370°C and 540°C. The pressure can be between 100 and 1000 psig (7.9 to 70 bar), but is preferably between 200 and 700 psig (14.8 to 49.3 bar). The liquid hourly space velocity is generally between 0.1 and 10 hr$^{-1}$, preferably between 0.5 and 4 hr$^{-1}$ and the hydrogen to hydrocarbon mole ratio is generally between 1 and 20, preferably between 4 and 12.

The catalyst can also be used for hydroisomerization of normal paraffins, when provided with a hydrogenation component, e.g. platinum. Hydroisomerization is carried out at a temperature between 90°C to 370°C, preferably 140°C to 290°C, with a liquid hourly space velocity between 0.01 and 2 hr$^{-1}$, preferably between 0.25 and 0.50 hr$^{-1}$, employing hydrogen such that the hydrogen to hydrocarbon mole ratio is between 1 and 5. Additionally, the catalyst can be used for olefin or aromatics isomerization employing temperatures between 0°C and 370°C.

The catalyst can also be used for reducing the pour point of gas oils. This process is carried out at a liquid hourly space velocity between about 0.1 and about 10 hr$^{-1}$ and a temperature between about 250°C and about 540°C and a pressure of between about 100 psig (7.9 bar) and about 2000 psig (138.9 bar).

Other reactions which can be accomplished employing the catalyst of this invention containing a noble metal, e.g. platinum, or a non-noble metal, e.g. nickel, tungsten, molybdenum, cobalt and combinations thereof, include hydrocracking, hydrogenation-dehydrogenation reactions and desulfurization reactions, olefin polymerization (oligomerization), and other organic compound conversions such as the conversion of alcohols (e.g. methanol) to hydrocarbons.

In the following Examples product crsytallinity is expressed as the ratio of the intensity of the reflection at two theta=22.5° to that of a standard sample of zeolite Beta.

Example 1

A 0.22 gram quantity of NaAlO$_2$ and 1.16 grams NaCl were dissolved in 36.7 grams deionized water. The resulting solution was mixed with 36.9 grams tetraethylammonium bromide. To this mixture was added 51.0 ml of 3.42 N tetraethylammonium hydroxide, with agitation. This solution was transferred to a 300 ml stainless steel autoclave. Twenty-one grams of commercial silica gel was then added to the solution and stirred vigorously in the autoclave for 2 minutes. The autoclave was then sealed and heated to 160°C, stirring at 400 rpm at autogenous pressure for 3 days. The reaction mixture had the composition, in mole ratios, as follows:

| | |
|---|---|
| SiO$_2$/Al$_2$O$_3$ | 400 |
| H$_2$O/SiO$_2$ | 10 |
| OH$^-$/SiO$_2$ | 0.50 |
| Na$^+$/SiO$_2$ | 0.10 |
| TEA$^+$/SiO$_2$ | 1.0 |

The final solid product was filtered from the reaction liquid upon termination of the reaction at 3 days, washed with water and dried at about 110°C.

The composition of the product, in weight percent was:

| | | |
|---|---|---|
| SiO$_2$ | = | 81.6 |
| Al$_2$O$_3$ | = | 0.35 |
| Na | = | not detected |
| C | = | 8.52 |
| N | = | 1.29 |
| Ash | = | 74.0 |
| SiO$_2$/Al$_2$O$_3$ mole ratio | = | 396 |

4

X-ray analysis proved the product to be 50% crystalline zeolite Beta having the characteristic lines set forth in Table 1.

Example 2

A 0.22 gram quantity of NaAlO₂, 1.9 grams of NaCl, and 36.9 grams of tetraethylammonium bromide were dissolved in 40.1 grams of deionized water. To this solution was added 47.6 ml of 3.66 N tetraethylammonium hydroxide. The resulting solution was added to 21.0 grams of commercial silica gel in a 300 ml stainless steel autoclave. The mixture, which had the same composition in mole ratios as that of Example 1, was stirred for 30 seconds, then the autoclave was sealed and stirring and heating begun.

This reaction mixture was heated at 160°C with stirring (400 rpm) for 65 hours before the autoclave was quenched in an ice bath to terminate crystallization.

The final solid product was processed as in Example 1. X-ray analysis of the product showed the material to be 80% crystalline zeolite Beta. The $SiO_2/Al_2O_3$ mole ratio of the product was 212.

Example 3

A 0.22 gram quantity of NaAlO₂, 1.2 grams of NaCl, and 36.9 grams of tetraethylammonium bromide were dissolved in 36.7 grams of deionized water. To this solution was added 51.0 ml of 3.44 N tetraethylammonium hydroxide. The resulting solution was mixed with 21.0 grams of HiSil silica gel (precipitated hydrated $SiO_2$ containing about 6 weight percent free $H_2O$ and 4.5 weight percent bound $H_2O$ of hydration and having a particle size of about 0.02 micron). The solution was then allowed to stand at room temperature for 5 days. The reaction mixture had the same composition in mole ratios as that of Example 1, save that the value of the ratio TEA/SiO₂ was 0.1.

After digestion period the hydrogel was transferred to a 300 ml stainless steel autoclave and reacted with stirring (400 rpm) at 160°C. The crystallization was terminated at 5 days.

The final solid product was processed as in Example 1. X-ray analysis of the product showed the material to be 90% crystalline zeolite Beta. It had a $SiO_2/Al_2O_3$ mole ratio of 156.

Example 4

A 0.32 gram quantity of NaAlO₂ and 1.26 grams of NaCl were dissolved in 41.2 grams of deionized water. To this solution was added 130.2 ml of 3.45 N tetraethylammonium hydroxide. The resulting solution was added to 104.2 grams of tetraethylorthosilicate in a 300 ml stainless steel autoclave. The reaction mixture had the composition, in mole ratios:

| | |
|---|---|
| $SiO_2/Al_2O_3$ | 400 |
| $H_2O/SiO_2$ | 10 |
| $OH^-/SiO_2$ | 0.90 |
| $Na^+/SiO_2$ | 0.05 |
| $TEA^+/SiO_2$ | 0.90 |

The mixture was reacted at 160°C with stirring at 400 rpm. Reaction was permitted to proceed for 24 hours.

X-ray analysis of this product proved it to be 90% crystalline zeolite Beta. It had a $SiO_2/Al_2O_3$ mole ratio of 106.

Example 5

A 1.5 gram quantity of NaAlO₂ was dissolved in 63.0 grams of deionized water. A second solution was produced by dissolving 15.6 grams of tetraethylammonium bromide in 60.2 ml of 3.42 N tetraethylammonium hydroxide, then mixing it with the first solution. The resulting solution was then added to 21.0 grams of HiSil silica gel in a 300 ml stainless steel autoclave. The reaction mixture had the composition, in mole ratios:

| | |
|---|---|
| $SiO_2/Al_2O_3$ | 60 |
| $H_2O/SiO_2$ | 15 |
| $OH^-/SiO_2$ | 0.60 |
| $Na^+/SiO_2$ | 0.05 |
| $TEA^+/SiO_2$ | 0.80 |

The autoclave was immediately sealed and stirring, and heating, begun. The hydrogel was reacted at 160°C with stirring at 400 rpm. Reaction was terminated after 2 days.

X-ray analysis of the product showed the material to be 70% crsytalline zeolite Beta. Its $SiO_2/Al_2O_3$ molar ratio was 43.

Example 6

A zeolite Beta composition in accordance with the invention was tested for hydroisomerization of hexadecane. Three different catalysts were tested to provide comparison. The first catalyst was a fully

5

# 0 164 939

crystalline zeolite Beta synthesized in accordance with US—A—3,308,069 and having a silica/alumina mole ratio of 30. The second catalyst was the dealuminized product of the fully crystalline first catalyst. Dealumination was conducted by extraction with acid in accordance with the method described in EP—A—95304. The third catalyst was the product of Example 1 hereof.

Prior to testing, each catalyst was exchanged to the ammonium form with 1M $NH_4Cl$ solution at 90° reflux for an hour followed by platinum (0.6 wt.%) exchange of the tetramine complex at room temperature overnight. The platinum-exchanged material was thoroughly washed, oven dried at 130°C and air calcined at 350°C.

The catalysts were then placed, in turn, in a reactor and contacted with hexadecane in the presence of hydrogen. The test temperature was maintained at 250—350°C, the pressure at 500 psig (34.5 bar), and the liquid hourly space velocity at 1 $hr^{-1}$. Product Iso-$C_{16}$ and conversion percentages were measured over the test. From the results presented in Table 2 it can be seen that the partially crystalline zeolite Beta composition performed essentially as effectively as the much more expensive dealuminized fully crystalline zeolite Beta, and significantly better than the fully crystalline Beta having a silica/alumina mole ratio of 30.

## TABLE 2

| Iso-$C_{16}$ at | First catalyst— 30/1 Beta | Second catalyst dealuminized Beta | Third catalyst— partially crystalline Beta |
|---|---|---|---|
| 40% Conversion | 39 | 40 | 40 |
| 60% Conversion | 50 | 60 | 57 |
| 80% Conversion | 59 | 78 | 72 |

## Claims

1. A method for synthesizing a zeolite Beta composition which comprises preparing a mixture containing sources of alkali metal (M) oxide, an oxide of aluminum, a tetraethylammonium (R) compound, an oxide of silicon and water and having a composition, in terms of mole ratios, within the following ranges:

| | |
|---|---|
| $SiO_2/Al_2O_3$ | 20—1000 |
| $H_2O/SiO_2$ | 5—200 |
| $OH^-/SiO_2$ | 0.10—2.0 |
| $M/SiO_2$ | 0.01—1.0 |
| $R/SiO_2$ | 0.10—2.0 |

and maintaining the mixture at a temperature of 90°C to 200°C for from 24 hours to 7 days for crsytallization of said zeolite Beta, and terminating crsytallization when the zeolite Beta product is from 30% to 90% crystalline.

2. A method according to claim 1 wherein said mixture has the composition, in terms of mole ratios:

| | |
|---|---|
| $SiO_2/Al_2O_3$ | 30—500 |
| $H_2O/SiO_2$ | 10—100 |
| $OH^-/SiO_2$ | 0.2—1.0 |
| $M/SiO_2$ | 0.05—0.20 |
| $R/SiO_2$ | 0.20—1.0 |

3. A method according to claim 1 or claim 2 wherein the tetraethylammonium compound is the hydroxide and/or halide.

4. A method according to any preceding claim wherein $SiO_2/Al_2O_3$ is greater than 200.

5. A method according to any preceding claim wherein $OH^-/SiO_2$ is at least 0.5.

6. A method according to any preceding claim which comprises separating said product from the reaction mixture after terminating crystallization.

7. A method according to any preceding claim wherein crystallization is terminated by quenching.

8. A method according to any preceding claim wherein said product is calcined at a temperature of 200°C to 600°C for from 1 to 48 hours.

9. A synthetic zeolite Beta composition conforming to the formula, expressed in terms of mole ratios of oxides in the anhydrous state:

(0.1 to 4) $R_2O$: (0.05 to 2) $M_{2/n}O$: (0.1 to 5) $Al_2O_3$: (100) $SiO_2$

wherein M is at least one alkali metal cation having the valence n, and R is a cation derived from a tetraethylammonium compound or mixtures thereof, which composition is 30% to 90% crystalline.

10. A composition according to claim 9 wherein the value of the mole ratio $SiO_2/Al_2O_3$ is greater than 100.

11. A composition according to claim 9 or claim 10 which has been subjected to ion exchange with one or more cations selected from hydrogen, hydrogen precursors, rare earth metals, and metals from Groups IIA, IIIA, IVA, IB, IIB, IIIB, IVB, VIB and/or VIII of the periodic Table of Elements.

12. A composition according to any of claims 9 to 11 which has been calcined.

13. A process for effecting catalytic conversion of an organic compound-containing feedstock which comprises contacting said feedstock under catalytic conversion conditions with a catalyst comprising the composition claimed in any of claims 9 to 12.

**Patentansprüche**

1. Verfahren zur Synthese einer Zusammensetzung von Zeolith-Beta, welches umfaßt: Herstellung einer Mischung, die Quellen eines Alkalimetall (M)-oxids, von Aluminiumoxid, einer Tetraethylammonium (R)-verbindung, von Siliciumoxid und Wasser umfaßt, und bezogen auf die Molverhältnisse eine Zusammensetzung innerhalb der folgenden Bereiche aufweist:

| | |
|---|---|
| $SiO_2/Al_2O_3$ | 20—1000 |
| $H_2O/SiO_2$ | 5—200 |
| $OH^-/SiO_2$ | 0,10—2,0 |
| $M/SiO_2$ | 0,01—1,0 |
| $R/SiO_2$ | 0,10—2,0 |

und Halten dieser Mischung bei einer Temperatur von 90°C bis 200°C 24 Stunden bis 7 Tage lang, um den Zeolith-Beta zu kristallisieren und Abschluß der Kristallisation, wenn das Produkt Zeolith-Beta von 30% bis 90% kristallin ist.

2. Verfahren nach Anspruch 1, worin die Mischung bezogen auf die Molverhältnisse die Zusammensetzung aufweist:

| | |
|---|---|
| $SiO_2/Al_2O_3$ | 30—500 |
| $H_2O/SiO_2$ | 10—100 |
| $OH^-/SiO_2$ | 0,2—1,0 |
| $M/SiO_2$ | 0,05—0,20 |
| $R/SiO_2$ | 0,20—1,0 |

3. Verfahren nach Anspruch 1 oder 2, worin die Tetraethylammoniumverbindung das Hydroxid und/oder Halogenid ist.

4. Verfahren nach einem der vorstehenden Ansprüche, worin $SiO_2/Al_2O_3$ größer als 200 ist.

5. Verfahren nach einem der vorstehenden Ansprüche, worin $OH^-/SiO_2$ mindestens 0,5 beträgt.

6. Verfahren nach einem der vorstehenden Ansprüche, welches die Abtrennung des Produktes aus der Reaktionsmischung nach dem Abschluß der Kristallisation umfaßt.

7. Verfahren nach einem der vorstehenden Ansprüche, worin die Kristallisation durch Abschrecken beendet wird.

8. Verfahren nach einem der vorstehenden Ansprüche, worin das Produkt 1 bis 48 Stunden lang bei einer Temperatur von 200°C bis 600°C kalziniert wird.

9. Synthetische Zusammensetzung von Zeolith-Beta, die, bezogen auf die Molverhältnisse der Oxide, im wasserfreien Zustand der Formel entspricht:

$$(0,1 \text{ bis } 4) \ R_2O: (0,05 \text{ bis } 2) \ M_{2/n}O: (0,1 \text{ bis } 5) \ Al_2O_3: (100) \ SiO_2$$

worin M zumindest ein Alkalimetallkation mit der Wertigkeit n ist und R ein Kation darstellt, das von einer Tetraethylammoniumverbindung oder Mischungen davon abgeleitet ist, dessen Zusammensetzung 30 bis 90% kristallin ist.

10. Zusammensetzung nach Anspruch 9, worin der Wert des Molverhältnisses $SiO_2/Al_2O_3$ größer als 100 ist.

11. Zusammensetzung nach Anspruch 9 oder 10, die einem Ionenaustausch mit einem oder mehreren Kationen unterzogen wurde, die aus Wasserstoff, Wasserstoffvorstufen, Metallen der Seltenen Erden und Metallen der Gruppen IIA, IIIA, IVA, IB, IIB, IIIB, IVB, VIB und/oder VIII des Periodensystems der Elemente ausgewählt sind.

12. Zusammensetzung nach einem der Ansprüche 9 bis 11, die kalziniert wurde.

13. Verfahren zur Durchführung einer katalytischen Umwandlung eines eine organische Verbindung enthaltenden Ausgangsmaterials, bei dem dieses Ausgangsmaterial bei katalytischen Umwandlungsbedingungen mit einem Katalysator in Kontakt gebracht wird, der die Zusammensetzung nach einem der Ansprüche 9 bis 12 umfaßt.

**Revendications**

1. Un procédé de synthèse d'une composition de zéolite bêta qui consiste à préparer un mélange contenant des sources d'oxyde de métal alcalin (M), un oxyde d'aluminium, un dérivé de tétraéthylammonium (R), un oxyde de silicium et de l'eau, et présentant une composition, en termes de rapports molaires, comprise dans les intervalles suivants:

| | |
|---|---|
| . $SiO_2/Al_2O_3$ | 20—1 000 |
| . $H_2O/SiO_2$ | 5—200 |
| . $OH^-/SiO_2$ | 0,10—2,0 |
| . $M/SiO_2$ | 0,01—1,0 |
| . $R/SiO_2$ | 0,10—2,0 |

et à maintenir le mélange à une température de 90 à 200°C pendant une durée de 24 heures à 7 jours en vue de la cristallisation de cette zéolite béta, et à terminer la cristallisation lorsque la zéolite béta obtenus à atteint une cristallinité de 30 à 90%.

2. Un procédé selon la revendication 1, dans lequel ce mélange présente la composition, en termes de rapports molaires:

| | |
|---|---|
| . $SiO_2/Al_2O_3$ | 30—500 |
| . $H_2O/SiO_2$ | 10—100 |
| . $OH^-/SiO_2$ | 0,2—1,0 |
| . $M/SiO_2$ | 0,05—0,20 |
| . $R/SiO_2$ | 0,20—1,0 |

3. Un procédé selon la revendication 1 ou la revendication 2, dans lequel le dérivé de tétraéthylammonium est l'hydroxyde et/ou un halogénure.

4. Un procédé selon l'une quelconque des revendications précédentes, dans lequel la rapport $SiO_2/Al_2O_3$ est supérieur à 200.

5. Un procédé selon l'une quelconque des revendications précédentes, dans lequel le rapport $OH^-/SiO_2$ est au moins égal à 0,5.

6. Un procédé selon l'une quelconque des revendications précédentes, qui consiste à séparer ce produit du mélange réactionnel après achèvement de la cristallisation.

7. Un procédé selon l'une quelconque des revendications précédentes, dans lequel on termine la cristallisation par trempe.

8. Un procédé selon l'une quelconque des revendications précédentes, dans lequel on calcine ce produit à une température de 200°C à 600°C pendant une durée de 1 à 48 heures.

9. Une composition de zéolite béta synthétique se conformant à la formule suivante, exprimée en termes de rapports molaires d'oxyde à l'état anhydre:

$$(0,1 \text{ à } 4) \ R_2O/(0,05 \text{ à } 2) \ M_{2/n}O/(0,1 \text{ à } 5) \ Al_2O_3/(100) \ SiO_2$$

dans laquelle:
M représente au moins un cation de métal alcalin présentant la valence n; et
R représente un cation provenant d'un dérivé de tétraéthylammonium ou de mélanges de ces dérivés, cette composition présentant une cristallinité de 30 à 90%.

10. Une composition selon la revendication 9, dans laquelle la valeur du rapport molaire $SiO_2/Al_2O_3$ est supérieure à 100.

11. Une composition selon la revendication 9 ou la revendication 10, qui a été soumise à un échange d'ions avec un ou plusieurs cations choisis parmi: hydrogène, des précurseurs d'hydrogène, des métaux des terres rares et des métaux des groupes IIA, IIIA, IVA, IB, IIB, IIIB, IVB, VIB et/ou VIII de la Classification Périodique des Eléments.

12. Une composition selon l'une quelconque des revendications 9 à 11, qui à été soumise à calcination.

13. Un procédé de mise en oeuvre d'une **conversion catalytique** d'une charge contenant un dérivé organique qui consiste à mettre cette charge, dans des conditions de conversion catalytique, au contact d'un catalyseur comprenant la composition revendiquée dans l'une quelconque des revendications 9 à 12.